(19)

**Europäisches Patentamt**
**European Patent Office**
**Office européen des brevets**

(11)   **EP 3 849 413 B1**

(12)   **EUROPEAN PATENT SPECIFICATION**

(45) Date of publication and mention
of the grant of the patent:
**06.11.2024   Bulletin 2024/45**

(21) Application number: **19778839.1**

(22) Date of filing: **13.09.2019**

(51) International Patent Classification (IPC):
**A61B 5/08** (2006.01)       **A61B 8/08** (2006.01)

(52) Cooperative Patent Classification (CPC):
**A61B 5/08; A61B 8/485**

(86) International application number:
**PCT/EP2019/074570**

(87) International publication number:
**WO 2020/053428 (19.03.2020 Gazette 2020/12)**

(54) **METHOD, DEVICE AND APPARATUS FOR MEASURING DIAPHRAGMATIC FUNCTIONAL PARAMETERS**

VERFAHREN, VORRICHTUNG UND EINRICHTUNG ZUR MESSUNG VON DIAPHRAGMA-FUNKTIONSPARAMETERN

PROCÉDÉ, DISPOSITIF ET APPAREIL DE MESURE DE PARAMÈTRES FONCTIONNELS DIAPHRAGMATIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:   **14.09.2018   EP 18306202**

(43) Date of publication of application:
**21.07.2021   Bulletin 2021/29**

(73) Proprietors:
• **Association Institut de Myologie**
**75013 Paris (FR)**
• **Institut National de la Santé et de la Recherche Médicale (INSERM)**
**75013 Paris 13 (FR)**
• **SORBONNE UNIVERSITE**
**75006 Paris (FR)**
• **ASSISTANCE PUBLIQUE, HOPITAUX DE PARIS**
**75004 Paris (FR)**

(72) Inventors:
• **BACHASSON, Damien**
**75012 Paris (FR)**
• **HOGREL, Jean-Yves**
**92120 Montrouge (FR)**
• **DRES, Martin**
**94240 L'Hay-les-Roses (FR)**

• **GENNISSON, Jean-Luc**
**95000 Cergy (FR)**
• **SIMILOWSKI, Thomas**
**92130 Issy-les-Moulineaux (FR)**

(74) Representative: **IPAZ**
**Bâtiment Platon**
**Parc Les Algorithmes**
**91190 Saint-Aubin (FR)**

(56) References cited:
**EP-A1- 2 889 004      EP-A1- 2 889 004**
**US-A1- 2018 036 033      US-A1- 2018 036 033**

• **G H MILLS ET AL: "Unilateral magnetic stimulation of the phrenic nerve.", THORAX, vol. 50, no. 11, 1 November 1995 (1995-11-01), GB, pages 1162 - 1172, XP055560423, ISSN: 0040-6376, DOI: 10.1136/thx.50.11.1162**
• **UDO ZIFKO ET AL: "Transcortical TRANSCORTICAL AND CERVICAL MAGN ETIC STIMULATION WITH RECORDING OF THE DIAPHRAGM", MUSCLE AND NERVE, vol. 19, 1 May 1996 (1996-05-01), pages 614 - 620, XP055560426**
• **D MASKILL ET AL: "MOTOR CORTICAL REPRESENTATION OF THE DIAPHRAGM IN MAN", JOURNAL OF PHYSIOLOGY, vol. 443, 1 January 1991 (1991-01-01), pages 105 - 121, XP055560427**

- G H MILLS ET AL: "Unilateral magnetic stimulation of the phrenic nerve.", THORAX, vol. 50, no. 11, 1 November 1995 (1995-11-01), GB, pages 1162 - 1172, XP055560423, ISSN: 0040-6376, DOI: 10.1136/thx.50.11.1162
- UDO ZIFKO ET AL: "Transcortical TRANSCORTICAL AND CERVICAL MAGN ETIC STIMULATION WITH RECORDING OF THE DIAPHRAGM", MUSCLE AND NERVE, vol. 19, 1 May 1996 (1996-05-01), pages 614 - 620, XP055560426
- D MASKILL ET AL: "MOTOR CORTICAL REPRESENTATION OF THE DIAPHRAGM IN MAN", JOURNAL OF PHYSIOLOGY, vol. 443, 1 January 1991 (1991-01-01), pages 105 - 121, XP055560427

## Description

### Field of the invention

The present invention relates to a method, a device and an apparatus for measuring diaphragmatic functional parameters of a diaphragm of a human or an animal. The present invention is in the field of medicine, biology and physiology. The present invention is non-invasive, may be apply to all type of populations and may be used to asses, inter alia, diaphragm effort, diaphragm function, diaphragm work and diaphragm dysfunctions.

The invention can be used as part of feedback loop for ventilatory support, exercise monitoring programs, physiotherapy and rehabilitation, and animal model for research programs. The invention can also be used from the moment a diaphragmatic dysfunction is suspected. The invention can be implemented on patient under assisted, spontaneous or volitional ventilation or during apnea.

### Background to the invention

Diaphragm is the principal muscle of respiration. Diaphragmatic dysfunctions are often benign but may result from affections processes that interfere with diaphragmatic innervation, contractile properties, or mechanical coupling to the chest wall. Dysfunctions of the diaphragm can be associated with the presence of disease, dyspnea, reduced exercise capacity, sleep disturbances and, in the more severe cases, have a negative impact on survival.

Prior art discloses a technic regarded as the gold standard for the assessment of diaphragm function. This technic relies on the measurement of transdiaphragmatic pressure (Pdi). Pdi is defined as the difference between pleural and abdominal pressures that are inferred from pressures in the lower esophagus (Pes) and stomach (Pga), respectively. The measurement of Pes and Pga is most frequently achieved by passing a pair of balloon catheters through the nose, following local anesthesia of the nasal mucosa and pharynx. Although Pdi is specific of diaphragm activation, it is invasive and indirectly reflects the intensity of diaphragm contraction.

Another prior art technic is the surface electromyography of the diaphragm (EMGdi). This technic is noninvasive but may be contaminated by other respiratory muscles (and cardiac artifacts) and exhibits limited validity for assessing diaphragm activity. G H Mills ET AL: "Unilateral magnetic stimulation of the phrenic nerve.",Thorax, vol. 50, no. 11, 1 November 1995 (1995-11-01), pages 1162-1172, discloses a prior art method and apparatus.

Therefore, prior art does not comprise any available technic for the assessment of diaphragmatic functional parameters which is accurate and noninvasive. The need of such a technic is critical.

## Summary of the invention

An object of the invention is to provide a noninvasive technic to assess at least one functional parameter of a diaphragm of a human or an animal.

The invention is defined in the independent claims.

The stimulation according to the invention may be defined as an artificial stimulation as opposed to a natural stimulation. The natural stimulation comprises spontaneous and volitional respiratory maneuvers. The stimulation according to the invention does not comprise assisted ventilation or life support.

The movement of one or more parts of the diaphragm may be a movement of the entire diaphragm.

The stimulation may be directly applied to the diaphragm. The stimulation directly applied to the diaphragm may generate a local motion of one or more parts the diaphragm. The local motion of one or more parts of the diaphragm generated by the stimulation may propagate through a part or through the entire diaphragm.

The artificial stimulation of the diaphragm may encompass muscular responses of the diaphragm.

The movement of one or more parts of the diaphragm generated by the stimulation may arise from the diaphragm contraction.

Functional parameters may be understood as parameters that can be monitor, notably over time, and that can be used to characterize diaphragm functioning.

The stimulation according to the invention may comprise one stimulus or several stimuli or periodic stimuli.

In this summary of the invention, the expression technical means may comprise, preferably only, at least one computer, a central and/or a calculation unit and/or a processing unit, an analog electronic circuit (preferably dedicated), a digital electronic circuit (preferably dedicated), and/or a microprocessor (preferably dedicated), and/or software means.

In this summary of the invention, the term "determining" may also be defined as assessing or estimating a parameter or a value.

According to a first variant of the method, the stimulation of the diaphragm may be an electrical or a magnetic stimulation.

The electrical and/or magnetic stimulation may be performed on the nervous system through:

- transcranial stimulation (supraspinal levels), and/or
- cervicomedullary (spinal) stimulation, and/or
- phrenic roots stimulation, and/or
- one or two of the main trunks of the phrenic nerve, and/or
- stimulation the diaphragm itself.

The electrical and/or magnetic stimulation may be applied during ventilation of the human or the animal. Namely, the human or the animal may be under spontaneous or volitional or respiratory maneuvers or under assisted ventilation or life support when the electrical and/or magnetic stimulation are applied to said human or animal.

According to the first variant, the processing step c) may further comprise a measure of the amplitude of the movement of the diaphragm. Preferably, the determination of the amplitude of the movement of the diaphragm may be based on an intensity of the stimulation.

According to the first variant, the determining step d) further may comprise a determination of a nerve conduction velocity.

The nerve conduction velocity may be observed based on the time separating the stimulation of the diaphragm from the occurrence of a movement of the diaphragm associated to said stimulation. A determination of a nerve conduction disorder is merely the observation of an anomaly of nerve conduction based on a nerve conduction delay.

According to the first variant, the determining step d) may comprise a determination of:

- a localized paralysis of the diaphragm.

Preferably, the determination of the contractility of the diaphragm, and/or a localized paralysis of the diaphragm, may be based on the propagation speed of a movement through the diaphragm.

The determination of the contractility of the diaphragm, and/or a localized paralysis of the diaphragm, may be based on:

- the propagation speed of a movement through the diaphragm, and/or
- the amplitude of the movement of the diaphragm, and/or
- a stiffness of the diaphragm.

The linear relation between the stiffness of the diaphragm and the propagation speed of a movement through the diaphragm, generated by the stimulation, may be used to determine the contractility.

The stiffness of one or more parts of the diaphragm, or of the entire diaphragm, may be determined based on the propagation speed of a movement through the diaphragm. The stiffness of one or more parts of the diaphragm, or of the entire diaphragm, may be determined based on the amplitude of the movement of the diaphragm.

A velocity profile of the diaphragm tissue is equivalent to a propagation of a movement from one or more parts of the diaphragm to one or more adjacent parts over time.

During the processing step c), the velocity profile of the diaphragm tissue may be processed, by technical means, to measure:

- peak heights being indicative of a contractility of the diaphragm, and/or
- peak areas being indicative of a contractility of the diaphragm, and/or
- peak widths being indicative of a contractility of the diaphragm, and/or
- peak slopes being indicative of a contractility of the diaphragm, and/or
- time to peaks being indicative of a contractility of the diaphragm, and/or
- an halftime relaxation being indicative of a contractility of the diaphragm, and/or
- an integral of diaphragm displacement within the inspiratory time over time being indicative of a diaphragmatic work, and/or
- a diaphragm displacement-time index computed as the product of a mean diaphragm displacement per breath within the inspiratory time and the ratio between an inspiratory time and the total respiratory cycle time being indicative of diaphragm function.

According to a second variant:

- the stimulation step a) may further comprise a mechanical stimulation and/or an acoustic stimulation of one or more parts of the diaphragm generating a mechanical wave and/or an acoustic wave at said given part, the mechanical wave and/or the acoustic wave propagating towards adjacent parts of said given part,
- the imaging step b) may further comprise imaging said given part and said adjacent parts through which the mechanical wave and/or the acoustic wave propagate(s).

According to the second variant, the mechanical wave and/or the acoustic stimulation may be an ultrasonic stimulation, said ultrasonic stimulation comprising an emission of one or more focused ultrasound waves per second towards a given part of the diaphragm, said one or more focused ultrasound waves generating an elastic shear wave at said given part, the elastic shear wave propagating towards adjacent parts of said given part.

The shear wave generated at a given part of the diaphragm is preferably a movement generated at said given part of the diaphragm.

Hence, the shear wave is preferably one of the parameters measured at step c) that are indicative of movement of one or more parts of the diaphragm.

Hence, the step c) may comprise the step of processing, by technical means, images previously acquired during the imaging step to measure:

• one or more shear wave of a given part of the diaphragm over time, and/or
• a propagation of a shear wave from said one or more parts of the diaphragm to one or more adjacent parts over time, and/or

- a propagation speed of the shear wave from said one or more parts of the diaphragm to one or more adjacent parts over time, and/or
- one or more shear wave of different parts of the diaphragm over time, and/or
- an amplitude of a shear wave of one or different parts of the diaphragm over time, and/or
- a time separating the stimulation of the diaphragm from the occurrence of a shear wave of the diaphragm associated to said stimulation.

According to the second variant, the processing step c) may further comprise a measure of a propagation velocity of the shear wave.

The propagation velocity of the shear wave is equivalent to a propagation of a movement from one or more parts of the diaphragm to one or more adjacent parts over time.

According to the second variant, the determining step d) may further comprise a determination of a contractility of the diaphragm. Preferably, the determination of the contractility of the diaphragm may be based on the propagation velocity of the shear wave.

A contractility of one or more parts of the diaphragm, or of the entire diaphragm, may be determined based on the propagation velocity of the shear wave.

The contractility of the diaphragm may be determined based on a stiffness of the diaphragm.

The linear relation between the stiffness of the diaphragm and the propagation velocity of the shear wave, generated by the stimulation, may be used to determine the contractility.

The stiffness of one or more parts of the diaphragm, or of the entire diaphragm, may be determined based on the propagation velocity of the shear wave.

According to the second variant, the stimulation step a) may be performed during ventilation of the human or the animal.

Namely, the human or the animal may be under spontaneous or volitional or respiratory maneuvers or under assisted ventilation or life support when the mechanical and/or acoustic stimulations is applied to said human or animal.

According to the second variant, the determining step d) may further comprise a determination of a diaphragm work.

According to the second variant, the determining step d) may further comprise a determination of a diaphragm activity. Preferably, the determining step d) is performed during ventilation of the human or the animal. Preferably, the determination of the diaphragm activity is based on the propagation velocity of the shear wave. Preferably, the determination of the diaphragm activity is based on the stiffness of the diaphragm. The linear relation between the stiffness of the diaphragm and the propagation velocity of the shear wave, generated by the stimulation, may be used to determine the diaphragm activity.

According to the second variant, the determining step d) may further comprise a determination of a transdiaphragmatic pressure. Preferably, the determining step d) is performed during ventilation of the human or the animal. Preferably, the determination of the transdiaphragmatic pressure is based on the variation of the propagation velocity of the shear wave. The linear relation between the transdiaphragmatic pressure and the propagation velocity of the shear wave, generated by the stimulation, may be used to determine the transdiaphragmatic pressure based on the sole value of the propagation velocity of the shear wave.

According to the second variant, the stimulation step a) may further comprise successive emissions of focused ultrasound waves towards the region of interest, each of said successive emissions being performed:

- according to a different axis, and/or
- according to a different focal length,

a determination of spatial organization of muscles fascicles.

Preferably, the determination of spatial organization of muscles fascicles may be based on three-dimensional velocity fields reconstruction.

According to the second variant, during the processing step c), images of diagram tissue previously acquired during the imaging step b), may be processed, by technical means, to measure the mechanical and/or acoustic wave propagating through said diaphragm tissue. A velocity profile of the mechanical and/or acoustic wave is equivalent to a propagation of a movement from one or more parts of the diaphragm to one or more adjacent parts over time.

During the processing step c), the velocity profile of the diaphragm tissue may be processed, by technical means, to measure:

- peaks heights of diaphragm stiffness being indicative of diaphragm contractility, and/or
- a frequency of stimulation being indicative of diaphragm contractility, and/or
- an integral of diaphragm stiffness within the inspiratory time over time being indicative of the work of the diaphragm, and/or
- a diaphragm stiffness-time index computed as the product of the diaphragm stiffness changes per breath within the inspiratory time and the ratio of the inspiratory time over the total respiratory time, being indicative of diaphragm function, and/or
- a slope of the profile being indicative of the contractility of the diaphragm.

The first and the second variants are not mutually exclusive unless otherwise specified.

The one or more moving parts of the diaphragm parts have been set in motion in response to a stimulation of the diaphragm, said stimulation having generating a movement of one or more parts of the diaphragm.

The means for processing may comprise, preferably only, at least one computer, a central and/or a calculation unit and/or a processing unit, an analog electronic circuit (preferably dedicated), a digital electronic circuit (preferably dedicated), and/or a microprocessor (preferably dedicated), and/or software means.

The functional parameters of the diaphragm comprise a contractility and/or a diaphragmatic pressure and/or a diaphragm effort and/or a diaphragm function and/or a diaphragmatic work and/or a nerve conduction velocity and/or a spatial organization of muscle fascicule(s).

The device according to the second aspect of the invention is arranged and/or configured and/or programmed to implement the method, or any step or feature of the method, according to the first aspect of the invention.

The means for processing may comprise, preferably only, at least one computer, a central and/or a calculation unit and/or a processing unit, an analog electronic circuit (preferably dedicated), a digital electronic circuit (preferably dedicated), and/or a microprocessor (preferably dedicated), and/or software means.

The means for stimulating may be arranged and/or configured and/or programmed to provide an electrical stimulation and/or a magnetic stimulation.

The means for processing may be arranged and/or configured and/or programmed to determine the amplitude of the movement of the diaphragm based on an intensity of the stimulation.

The means for processing may be arranged and/or configured and/or programmed to determine a nerve conduction velocity.

The means for processing may be arranged and/or configured and/or programmed to determine:

- a contractility of the diaphragm, and/or
- a localized paralysis of the diaphragm.

Preferably, based on a propagation speed of a movement through the diaphragm, the means for processing may be arranged and/or configured and/or programmed to determine:

- a contractility of the diaphragm, and/or
- a localized paralysis of the diaphragm.

The means for stimulating may be arranged and/or configured and/or programmed to provide a mechanical stimulation and/or an acoustic stimulation of one or more parts of the diaphragm to generate a mechanical wave and/or an acoustic wave at said given part, the mechanical wave and/or the acoustic wave propagating towards adjacent parts of said given part, and

- the means for imaging may be arranged and/or configured and/or programmed to image said given part and said adjacent parts through which the mechanical wave and/or the acoustic wave propagate(s).

The means for stimulating may be arranged and/or configured and/or programmed to provide an ultrasonic stimulation, said ultrasonic stimulation comprises an emission of one or more focused ultrasound waves per second towards a given part of the diaphragm, said one or more focused ultrasound waves generate an elastic shear wave at said given part, the elastic shear wave propagates towards adjacent parts of said given part.

The means for imaging may be arranged and/or configured and/or programmed to measure a propagation velocity of the shear wave.

The means for processing may be arranged and/or configured and/or programmed to determine a contractility of the diaphragm based on the propagation velocity of the shear wave.

The apparatus may be arranged and/or configured and/or programmed to measure one or more diaphragmatic functional parameters of a diaphragm of a human or an animal during ventilation of the human or the animal.

The means for processing may be arranged and/or configured and/or programmed to determine a diaphragm work.

The means for processing may be arranged and/or configured and/or programmed to determine a diaphragm activity based on the propagation velocity of the shear waves.

The means for processing may be arranged and/or configured and/or programmed to determine a transdiaphragmatic pressure based on the variation of the propagation velocity of the shear wave.

The means for stimulating may be arranged and/or configured and/or programmed to emit successive focused ultrasound waves towards the region of interest:

- according to a different axis, and/or
- according to a different focal length,

the means for processing may be arranged and/or configured and/or programmed to determine a spatial organization of muscles fascicule(s). Preferably, the determination of spatial organization of muscles fascicles may be based on three-dimensional velocity fields reconstruction.

The functional parameters of the diaphragm comprise a contractility and/or a diaphragmatic pressure and/or a diaphragm function and/or a diaphragm effort and/or a diaphragmatic effort and/or a diaphragmatic work and/or a nerve conduction velocity and/or a spatial organization of muscle fascicule(s).

The apparatus according to the third aspect of the invention is arranged and/or configured and/or programmed to implement the method, or any step or feature of the method, according to the first aspect of the invention.

## Brief description of the drawings

Further objects, features and advantages will appear from the following detailed description of several embodiments of the invention with references to the drawings, in which:

- FIGURE 1 is a schematic representation of the experimental setup used on the human participants,
- FIGURE 2 is a schematic representation of responses of a diaphragm to an electrical or magnetic stimulation,
- FIGURES 3 and 4 are respectively images of diaphragmatic displacements over time in response to an electrical stimulation of high and respectively low intensity,
- FIGURES 5 and 6 are respectively graphs illustrating the amplitude of diaphragmatic displacements according to the intensity of an electrical stimulation and respectively a magnetic stimulation,
- FIGURE 7 shows a set of graphs illustrating a set of measurements performed during static inspiratory efforts against closed airways,
- FIGURE 8 shows a set of graphs illustrating a set of measurements performed during ventilation against inspiratory loading,
- FIGURE 9 shows a set of graphs, each graph is from a different participant, of individual data points illustrating relationship between transdiaphragmatic pressure and diaphragm shear modulus measures from ultrafast ultrasound image in response to an ultrasonic stimulation during submaximal static inspiratory efforts against closed airways,
- FIGURE 10 shows a set of graphs, each graph is from a different participant, of individual data points illustrating relationship individual data points illustrating relationship between transdiaphragmatic pressure and diaphragm shear modulus measures from ultrafast ultrasound image in response to an ultrasonic stimulation during unloaded ventilation and ventilation against inspiratory loading,
- FIGURE 11 shows hysteresis curve between transdiaphragmatic pressure and diaphragm shear modulus measures from ultrafast ultrasound image in response to an ultrasonic stimulation during ventilation against inspiratory loading in one participant.

## Detail description of embodiments of the invention

The embodiments hereinafter described are not restrictive, other embodiments comprising a selection of features described hereinafter may be considered. A selection may comprise features isolated from a set of features (even if this selection is isolated among a sentence comprising other features thereof), if the selection is sufficient to confer a technical advantage or to distinguish the invention form the state of the art. This selection comprises at least a feature, preferably described by its technical function without structural features, or with a part of structural details if this part is sufficient to confer a technical advantage or to distinguish the invention form the state of the art on its own.

Furthermore, the embodiments hereinafter are non-limitative embodiments that are within the scope of the above summary of the invention. Thus, any isolated feature of the below embodiments is considered in combination with the more general or functional steps or features of the above summary of the invention.

## Participants

A total of fifteen healthy participants were studied. All participants gave written informed consent.

## Experimental setup

Participants were studied in a semirecumbent position (40 degrees) with uncast abdomen, breathing through a mouthpiece while wearing a nose clip. The mouthpiece was connected to a three-way valve and pneu-motachograph for flow measurement. Mouth pressure (Pmo) was recorded using a differential transducer. Pressure in the lower esophagus (Pes) and pressure in stomach (Pga) were measured using 10-$^{cm}$ balloon catheters, connected separately to differential pressure transducers (model DP45-30; Validyne, Northridge, CA) as previously described. Flow and pressures signals were digitized (Powerlab, ADInstruments, Sydney, Australia) and recorded at a sampling frequency of 2 kHz (Labchart, ADInstruments). Transdiaphragmatic pressure (Pdi) was obtained by online subtraction of Pes from Pga. Ultrasound measurements. Diaphragm ultrasound imaging and shear wave elastography were performed using an Aixplorer Ultrasound scanner (V9.2, Supersonic Imagine, Aix-en-Provence, France) driving a 10-$^2$ MHz linear transducer array (SL10-$^2$, Supersonic Imagine). Settings were defined as follow: B-mode enabled; supersonic shear imaging mode enabled; penetration mode enabled; tissue tuner at 1540 m·s-$^1$; dynamic range at 80 dB; Gain and time gain compensation were tailored for each patient. Scale for Shear Wave Elastography (SWE) was adjusted if required. Sampling rates for B-mode imaging and SWE were 12 and 2 Hz, respectively. A generous amount of water-soluble transmission gel was used during scanning for optimal acoustic coupling and minimal pressure was applied to the transducer in order to limit tissue deformation and modification of ventilator mechanics. The diaphragm was scanned at the right zone of apposition, on the posterior axillary line vertical to the chest wall at the 9th-11th intercostal space. The rotation and angle of the transducer was the finely adjusted to obtain maximal echo intensity from diaphragmatic pleural and peritoneal peritonea. Ultrasound scanned were triggered by the Powerlab for synchronizing ultrasound, flow, and pressures recordings. The transducer position was marked on the skin. Ultrasound measurements were performed by a trained intensivist (MD).

Study protocol

The study was carried out as follows: i) measurement of maximal static inspiratory pressure (PImax), ii) recordings during apnea at functional residual capacity (FRC), iii) recordings during inspiratory efforts against closed airways, iv) recordings during ventilation against inspiratory loading.

*Maximal static inspiratory pressure*

PImax was measured at FRC. At least five trials were performed until three reproducible efforts, with less than 10% variance, were obtained. Maximal Pmo generated amongst the three reproducible trials was defined as PImax.

*Apnea at FRC and static inspiratory efforts against closed airways*

During these tasks, the mouthpiece was disconnected from the three-way valve and flow was not monitored. Pressures and Shear Modulus of the diaphragm (SMdi) were measured during about 5s apnea and during inspiratory efforts against closed airways at 10, 20, 30, 40, 50, and 60 % of PImax (two participants did not performed 60% PImax). Both apnea and inspiratory efforts were performed at FRC. Participant (n=13) were asked to reach progressively the target Pmo and to maintain their effort during about 10s. Visual feedback of generated Pmo and guidelines were provided to participants using the built-in software option. Each task was repeated twice. Tasks were alternated with 1-2 min of unloaded breathing.

*Ventilation against inspiratory loading*

An in-house developed apparatus was used to perform ventilation against inspiratory elastic loads. Briefly, the device consisted of a cylindrical adjustable pressure chamber connected to a non-rebreathing valve. The negative pressure was generated by a commercially available vacuum cleaner. Pressure in the chamber (Pch) was measured continuously using a differential pressure transducer. The dead space of the device was estimated at about 600 ml. Participants (n=15) underwent a stepwise inspiratory loading protocol at 10, 20, 30, 40 and 50% of PImax (two participants (5, 10) did not performed 50% PImax, one participant (10) did not performed 40% PImax, one participant (7) also performed 60% PImax). Each task was repeated twice. During each task, at least six regular respiratory cycles were recorded. Tasks were alternated with 1-2 min of unloaded breathing.

Data analysis

Pes, Pga, Pdi, Pmo, Pch and flow were analyzed offline using standardized scripts in MATLAB (Mathworks, Natick, MA, USA). Frames from Bmode and SWE recordings were exported using the ultrasound scanner research pack (Soniclab, v11, Supersonic imagine) and processed using standardized scripts in MATLAB (Mathworks). SMdi was calculated assuming a linear elastic behavior in muscle tissue (4) as $SMdi = \rho \cdot Vs^2$ where $\rho$ is the density of muscle ($1000$ $kg \cdot m^{-3}$), and Vs is the shear wave speed or the propagation velocity of the shear wave in $m \cdot s^{-1}$. The Young Modulus E, and so the tissue elasticity, for soft tissues can be considered as being equal to $E = 3 \cdot SMdi$. A rectangular region of interest was manually defined on the first frame of each stack as large as possible between the diaphragm pleural and parietal peritonea. For static inspiratory efforts measurements, signals were manually selected when Pmo was stabilized at the targeted levels. Pressures and SMdi where averaged over the duration of the selected period. Coefficients of variation were computed to assess variability of pressures and SMdi within the selected period. During ventilation against inspiratory loading, maximal SMdi and pressures swing during inspiratory time were computed for each cycle. Cycles were discarded in the case of loss of diaphragm visualization and SMdi > 90 kPa (e.g. when the ROI was in a rib instead of diaphragm). Ultimately, 66 were discarded over 970 recorded cycles. Mean SMdi value during apnea at FRC was subtracted from average and maximal SMdi during static efforts and loaded ventilation, respectively.

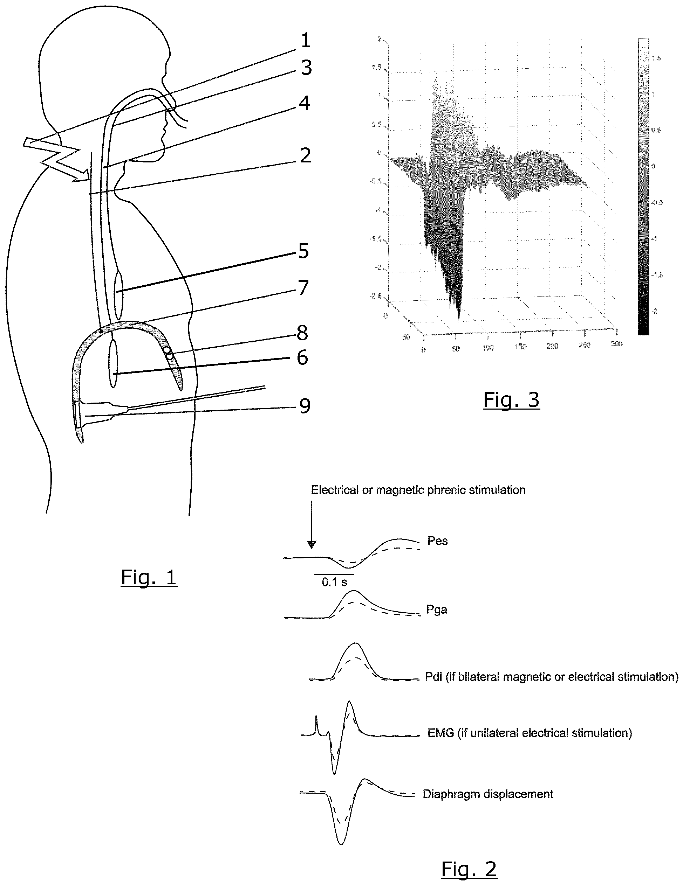

FIGURE 1 shows the schematic representation of the experimental setup used for each participant for a first and a second embodiment. The setup comprises an electrode (not represented) for the electrical or the magnetic stimulation 1 of the phrenic nerve 2. According to the setup the phrenic roots is stimulated in the region of the neck but other regions and/or arrangements may be used.

In order to performed Pdi measurement and correlate Pdi measurement to measurements according to the invention, an esophageal balloon catheter 3 and a gastric balloon catheter 4 are used for Pdi measurements. The balloon in the esophagus 5 located above the diaphragm 7 and the balloon in the stomach 6 under the diaphragm 7 are also illustrated. This invasive protocol is required for Pdi measurements which is the current gold standard for the assessment of diaphragmatic functional parameters. This invasive protocol is not part of the invention but was carried out to prove that the invention provides reliable results and is an alternative to Pdi measurements.

Surface AgCl/Ag electrodes 8 are located on the outer wall of the chest wall about the diaphragm 7 for EMG measurements. The ultrasound probe 9, uses for ultrafast imaging of the diaphragm 7 and stimulating the diaphragm 7, is positioned on the outer wall of the chest wall and moved on any desired location as needed.

The method that comprises emitting at least 100 unfocused ultrasound waves per second, detecting ultrasound waves reflected and/or scattered by organic tissues of the human or the animal located in the region and processing the reflected and/or scattered ultrasound waves over time to generate images is known by ultrafast ultrasound imaging. The apparatus and the setting uses for ultrafast ultrasound imaging are described above.

According to the experiment and as necessary, the processing of ultrasound images is used to measure one or more movements of a given part of the diaphragm over time, and/or a propagation of a movement from said one or more parts of the diaphragm to one or more adjacent parts over time, and/or a propagation speed of a movement from said one or more parts of the diaphragm to one or more adjacent parts over time, namely the SMdi, and/or one or more movements of different parts of the diaphragm over time, and/or an amplitude of a movement of one or different parts of the diaphragm over time, and/or a time separating the stimulation of the diaphragm from the occurrence of a movement of the diaphragm associated to said stimulation. Software, settings and parameters used for measurements, data analysis and determination are described above.

According to the first embodiment illustrating through figures 2 to 6, the stimulation of diaphragm in order to generate a movement of one or more parts of the diaphragm was carried out through electrical or magnetic stimulation 1. This embodiment exhibits great advantages because is the only one that is non-volitional and that can be carried out when the patient is under assisted ventilation and/or under life-support. This embodiment is also the only one that can be carried out when the patient show disorders that prohibit volitional ventilation (or apnea) or simply cannot achieve required respiratory maneuvers.

FIGURE 2 shows a schematic representation of responses of a diaphragm to an electrical or magnetic stimulation 1. The dash line corresponds to the lower stimulation intensity apply and the plain line to the higher stimulation intensity apply. One can see the effects on the Pes, the Pga, the Pdi (if bilateral magnetic or electrical stimulation 1), the EMG (if unilateral electrical stimulation 1) and the diaphragm 7 displacement over time elicited by the electrical or magnetic stimulation 1. One can see the clear differences of the responses for each measured parameters depending on the stimulation intensity.

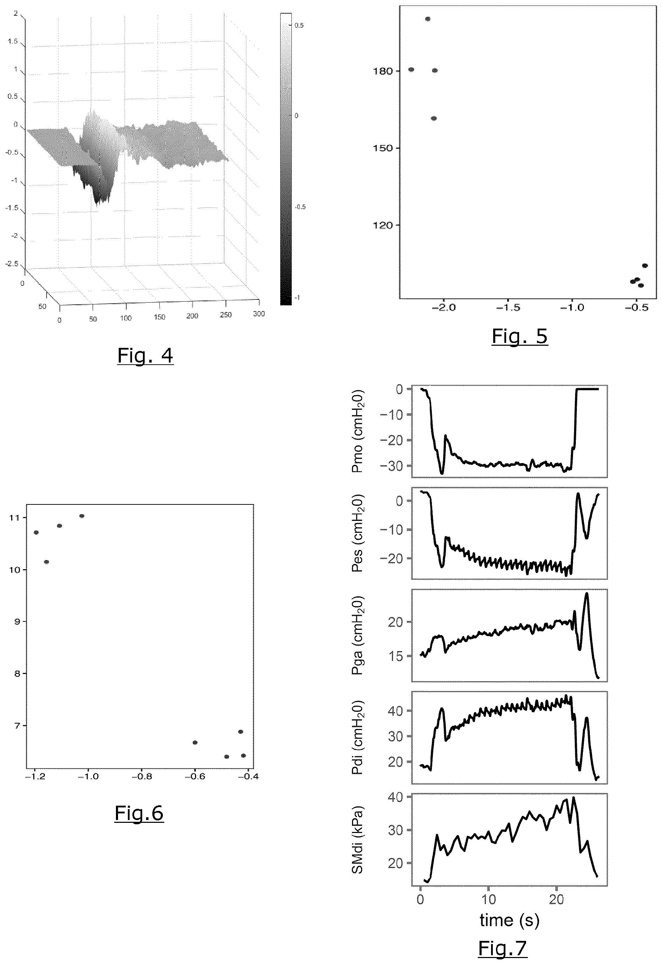

Figures 3 and 4 show diaphragmatic displacements in millimeters (mm), in Y-axis, over time in milliseconds (ms), in X-axis, according to ultrasonic piezo transducers, in Z-axis. Figure 3 shows the image of diaphragmatic displacements amplitude in millimeters over time in response to a supramaximal electrical stimulation. Figure 4 shows the image of diaphragmatic displacements amplitude in micrometers over time in response to an electrical stimulation selected to elicit half the EMG response obtain with supramaximal stimulation. Similar results were obtained through magnetic stimulations 1. These results clearly show that the amplitude of the diaphragm 7 displacements in response to an electrical or magnetic stimulation 1 is linearly correlates to the intensity of the stimulation 1 applied.

A participant has been subjected to supramaximal electrical stimulations 1 and to electrical stimulations selected to elicit half the EMG response obtain with supramaximal stimulation. On figure 5 is shown a graph illustrating amplitudes of diaphragmatic displacements in mm, in X-axis, according to the intensity of the electrical stimulation 1 in millivolts (mV), in Y-axis. The amplitude of diaphragmatic displacements is comprised between 2 and 2.2 mm for a supramaximal electrical stimulation 1 and between 0.45 and 0.55 for electrical stimulation 1 selected to elicit half the EMG response obtain with supramaximal stimulation.

A participant has been subjected to supramaximal magnetic stimulations 1 and to magnetic stimulations selected to elicit half the Pdi response obtain with supramaximal stimulation. Figure 6 shows a graph illustrating amplitudes of diaphragmatic displacements in mm, in X-axis, according to the Pdi. The amplitude is comprised between 1 et 1.2 mm for a supramaximal magnetic stimulation 1 and between 0.45 et 0.55 for magnetic stimulation 1 selected to elicit half the Pdi response obtain with supramaximal stimulation.

The representation of figure 2 also illustrates the determination of the presence of nerve conduction velocity. A delay between the electrical or magnetic stimulation and the displacement of the diaphragm may be measured. This measurement of nerve conduction delay is relative to phrenic nerve conduction velocity.

Thus, among others, the main functional parameter that may be determined from the measurements elicited by such stimulations is the contractility of the diaphragm 7. The contractility of the diaphragm 7 can be assessed based on the intensity of the stimulation 1 from the moment it has established that a linear relation between the intensity stimulation 1 and the amplitude of the movement exists.

According to the second embodiment illustrated through figures 7 to 11, the stimulation of diaphragm in order to generate a movement of one or more parts of the diaphragm was carried out through ultrasonic stimulation. The method that comprises an ultrasonic stimulation comprising an emission of one or more focused ultrasound waves per second towards a given part of an elastic tissue for generating an elastic shear wave that propagates towards adjacent parts of said given part of the elastic tissue in order to observed the shear wave propagation is known as Shear Wave Elastography (SWE). SWE allows measuring the SMdi of a part or the diaphragm or the mean SMdi of the entire diaphragm. The apparatus and the setting used to perform SWE are described above.

FIGURE 7 shows a set of graphs illustrating a set of measurements performed during static inspiratory efforts against closed airways. Pmo, Pes, Pga, Pdi and SMdi were measured during static inspiratory efforts against closed airways. Mean selection duration for averaging data was 8.7 s (SD 3.9). Within selected data, mean of coefficient of variation for Pmo, Pes, Pga, Pdi, and SMdi were 14.2, 9.0, 6.3, 5.4, and 16.2 %, respectively. A clear linear relationship between mean Pdi swing and mean SMdi during all tasks is highlighted and identified. Mean Pdi significantly correlated to mean SMdi in all participants except one (r ranged from 0.60 to 0.92; in participants with significant correlation, all p were < 0.05; R = 0.76, 95% CIs [0.69, 0.82], p < 0.001, r was > 0.70 in ten over thirteen participants).

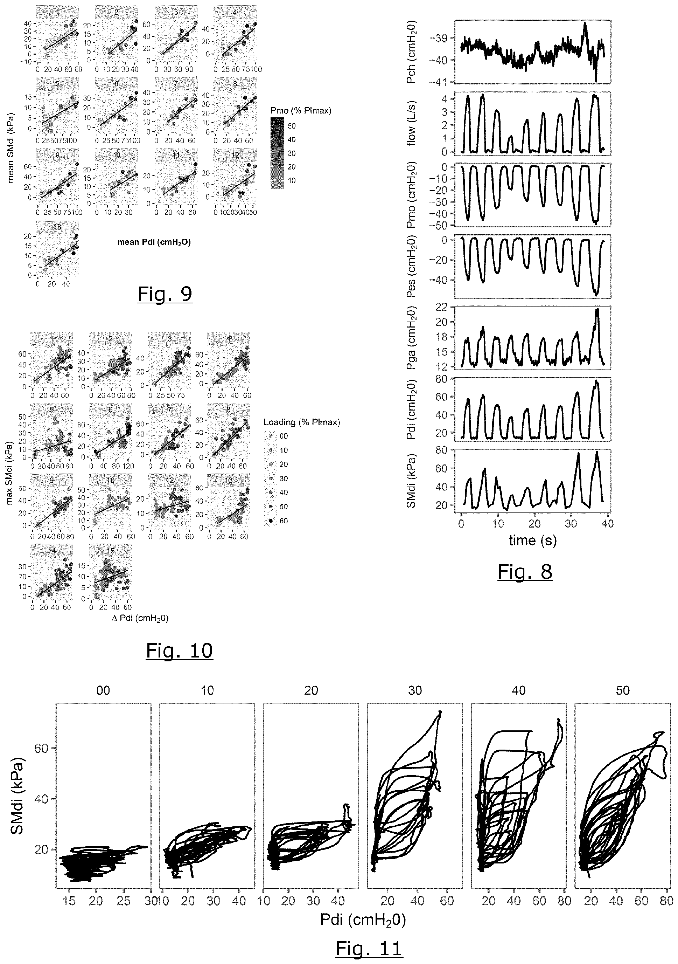

FIGURE 8 shows a set of graphs illustrating a set of measurements performed during ventilation against inspiratory loading. Pch, air flow, Pmo, Pes, Pga, Pdi and SMdi were measured during ventilation against inspiratory loading. Number of cycles analyzed per loading level was 11.8 (SD 3.0). A clear linear relationship between Pdi swing and maximal SMdi for all analysed cycles and all loading tasks is highlighted and identified. Maximal SMdi correlated to Pdi swing in all participants (r ranged from 0.32 to 0.95, all p < 0.01; R = 0.71, 95% CIs [0.68, 0.74], p < 0.001, r was > 0.70 in nine over fifteen participants).

The results illustrate on figure 7 and 8 show that increasing the inspiratory load during both static inspiratory efforts and ventilation against inspiratory loading resulted in an increase in Pdi. These results show strong linear relationship between max SMdi and Pdi swing during ventilation against inspiratory loading. These findings demonstrate for the first time that diaphragm activity can be noninvasively monitored using SWE during breathing.

Thus, among others, the main functional parameter that may be determined is the contractility of the diaphragm 7. The contractility of the diaphragm 7 can be assessed based on propagation velocity of the shear wave from the moment a linear relation between Pdi and SMdi is established.

FIGURES 9 and 10 illustrate, for a set of different participants, the relationship between mean Pdi and mean SMdi for different Pmo, and respectively the relationship between the amplitude of the Pdi according to maximum SMdi for different inspiratory loading. These results demonstrate that diaphragm stiffening is strongly related to the level of diaphragm activation as assessed by standard Pdi measurements. Moreover, high individual correlation coefficients between SMdi and Pdi have been observed in most participants. Slight offset of transducer angle in reference to the direction of muscle fascicles is known to reduce shear modulus value. Therefore, quality criteria for SMdi measurements must be established and adjustment of transducers in the three-dimensional space shall be assisted programmati-cally to obtain largest SMdi changes during ventilation. Muscle fascicles in reference to the probe might also be estimated using three dimensional SWE measurements.

FIGURE 11 shows hysteresis curves between transdiaphragmatic pressure and diaphragm shear modulus measures from ultrafast ultrasound images in response to an ultrasonic stimulation during ventilation against inspiratory loading in one participant. Figure 11 shows a set of graphs, each graphs corresponds to an inspiratory load, from left to right 0, 10, 20, 30, 40, 50, and 60 % of PImax.

Thus, among others, functional parameters that may be determined are the diaphragm work and the diagram activity.

SMdi coupled with functional respiratory investigations may help to detect diaphragm dysfunction. It might be particularly useful for detecting diaphragm hemi-paralysis. Diaphragm SWE might also particularly relevant within spontaneous breathing trials and/or pressure support ventilation in ventilated patients during the weaning phase. Diaphragm stiffening-time index may also be computed during spontaneous breathing trial.

Three examples of determination of functional parameters are set forth herein below. Each functional parameter has been determined form the data derived from the study described above and from which the figures 2 to 11 has been plotted.

As a first example, the transdiaphragmatic pressure (Pdi) is determined from the shear modulus (SMdi) measured in response to an ultrasonic stimulation. The shear modulus (SMdi) is determined from the following equation:

$$Pdi = a.SMdi + b,$$

where Pdi is in $cmH_2O$, SMdi is in kPa, and
a is equal to -22.77 in this example, preferably lower than -5 and/or higher than -40,
b is equal to 1.34 in this example, preferably lower than 5 and/or higher than 0.5.

For the latter model: degree of freedom was 161, p-value was < 0.0001, R-squared was 0.671, adjusted R-squared was 0.669, and F-statistics was 328.5.

As a second example, the transdiaphragmatic pressure (Pdi) is determined from a local displacement of the diaphragm (DISPdi), in other words a movement of a given part of the diaphragm, measured in response to cervical magnetic stimulation. The transdiaphragmatic pressure (Pdi) is determined from the following equation:

$$Pdi = c.DISPdi,$$

where Pdi is in $cmH_2O$, DISPdi is in mm, and
c is equal to 13.3 in this example, preferably lower than 30 and/or higher than 1.

For the latter model: degree of freedom was 67, p-value was < 0.0001, R-squared was 0.683, adjusted R-squared was 0.679, and F-statistics was 144.5.

As a Third example, a diaphragm compound muscle action potential (CMAPdi) is determined from a local displacement of the diaphragm (DISPdi), in other words a movement of a given part of the diaphragm, measured in response to a unilateral phrenic electrical stimulation. The compound muscle action potential (CMAPdi) of the diaphragm is determined from the following equation:

$$CMAPdi = d.SMdi,$$

where CMAPdi is in mV, DISPdi is in mm, and
d is equal to 118.5 in this example, preferably lower than 300 and/or higher than 20.

For the latter model: degree of freedom was 72, p-value was < 0.0001, R-squared was 0.556, adjusted R-squared was 0.550, and F-statistics was 90.06.

The invention is not restricted to embodiments described above and numerous adjustments may be achieved within the scope of the invention.

Moreover, features, alternatives and embodiments of the invention may be associated if they are not mutually exclusive of each other.

Thus, in combinable alternatives of previous embodiments:

- the measurement of nerve conduction delay may be performed through any stimulation eliciting a diaphragm contraction, and/or
- according to the first embodiment of the invention, functional parameter that may be determined from the measurements elicited by electrical and/or magnetic stimulations is: the diaphragmatic pressure and/or the diaphragm function and/or the diaphragmatic work, and/or
- according to the first embodiment of the invention, the velocity profile of the diaphragm tissue may be processed, by technical means, to measure:

  • peak heights being indicative of a contractility of the diaphragm, and/or
  • peak areas being indicative of a contractility of the diaphragm, and/or
  • peak widths being indicative of a contractility of the diaphragm, and/or
  • peak slopes being indicative of a contractility of the diaphragm, and/or
  • time to peaks being indicative of a contractility of the diaphragm, and/or
  • an halftime relaxation, and/or
  • an integral of diaphragm displacement within the inspiratory time over time being indicative of a diaphragmatic work, and/or
  • a diaphragm displacement-time index computed as the product of a mean diaphragm displacement per breath within the inspiratory time and the ratio between an inspiratory time and the total respiratory cycle time being indicative of diaphragm function, and/or

- according to the second embodiment, the ultrasonic stimulation may be substitute by any mechanical and/or acoustic stimulation, and/or
- according to the second embodiment, functional parameter that may be determined from the measurements elicited by mechanical and/or acoustic stimulation is: the diaphragmatic pressure and/or the diaphragm function and/or the diaphragmatic work, and/or
- according to the second embodiment, the velocity profile of the diaphragm tissue may be processed, by technical means, to measure:

  • peaks heights of diaphragm stiffness being indicative of diaphragm contractility, and/or
  • a frequency of stimulation being indicative of diaphragm contractility, and/or
  • an integral of diaphragm stiffness within the inspiratory time over time being indicative of the work of the diaphragm, and/or
  • a diaphragm stiffness-time index computed as the product of the diaphragm stiffness changes within the inspiratory time and the ratio of the inspiratory time over the total respiratory time, being indicative of diaphragm function, and/or
  • a slope of the profile being indicative of the contractility of the diaphragm.

**Claims**

1. Method for measuring one or more diaphragmatic functional parameters of a diaphragm (7) of a human or an animal among a contractility and/or a diaphragmatic pressure and/or a diaphragm function and/or diaphragm effort and/or a diaphragmatic work and/or an identification of the spatial organization of muscle fascicule(s), said method comprising the steps consisting of:

   - a) a stimulation step by which a stimulation of the diaphragm is provided to generate a movement of one or more parts of the diaphragm, said stimulation step does not comprise assisted ventilation or life support,
   - b) during the movement of said one or more parts of the diaphragm, imaging one or more parts of the diaphragm over time through an imaging step comprising the steps of:

     • emitting at least 100 unfocused ultrasound waves per second towards a region of the human or the animal comprising the one or more parts of the diaphragm to be imaged during the imaging step,

• detecting ultrasound waves reflected and/or scattered by organic tissues of the human or the animal located in the region,

• processing the reflected and/or scattered ultrasound waves over time to generate images by technical means,

- c) processing, by technical means, images previously acquired during the imaging step to measure:

• one or more movements of a given part of the diaphragm over time, and/or

• a propagation of a movement from said one or more parts of the diaphragm to one or more adjacent parts over time, and/or

• a propagation speed of a movement from said one or more parts of the diaphragm to one or more adjacent parts over time, and/or

• one or more movements of different parts of the diaphragm over time, and/or

• an amplitude of a movement of one or different parts of the diaphragm over time

- d) based on one or more measurements of the processing step, determining, by technical means, one or more functional parameters of the diaphragm from a linear relation between one of the parameters measured at step c) being indicative of movement of one or more parts of the diaphragm and the considered functional parameter.

2. Method according to claim 1, wherein the stimulation of the diaphragm is an electrical and/or a magnetic stimulation.

3. Method according to claim 2, wherein the processing step c) further comprises a measure of the amplitude of the movement of the diaphragm based on an intensity of the stimulation.

4. Method according to claim 2 or 3, wherein the determining step d) further comprises a determination of a nerve conduction velocity.

5. Method according to any of claims 2 to 4, wherein, based on a propagation speed of a movement through the diaphragm, the determining step d) comprises a determination of:

- a contractility of the diaphragm, and/or
- a localized paralysis of the diaphragm.

6. Method according to claim 1, wherein:

- the stimulation step a) further comprises a mechanical stimulation and/or an acoustic stimulation of one or more parts of the diaphragm generating a mechanical wave and/or an acoustic wave at said given part, the mechanical wave and/or the acoustic wave propagating towards adjacent parts of said given part,

- the imaging step b) further comprises imaging said given part and said adjacent parts through which the mechanical wave and/or the acoustic wave propagate(s).

7. Method according to claim 6, wherein the mechanical wave and/or the acoustic stimulation is an ultrasonic stimulation, said ultrasonic stimulation comprising an emission of one or more focused ultrasound waves per second towards a given part of the diaphragm, said one or more focused ultrasound waves generating an elastic shear wave at said given part, the elastic shear wave propagating towards adjacent parts of said given part.

8. Method according to claim 6 or 7, wherein the processing step c) further comprises a measure of a propagation velocity of the shear wave.

9. Method according to any of claims 6 to 8, wherein the determining step d) further comprises a determination of a contractility of the diaphragm based on the propagation velocity of the shear wave.

10. Method according to any of claims 6 to 9, wherein the stimulation step a) is performed during ventilation of the human or the animal.

11. Method according to any of claims 6 to 10, wherein the determining step d) further comprises a determination of a diaphragm work.

12. Method according to any of claims 6 to 11, wherein the determining step d) further comprises a determination of a diaphragm activity based on the propagation velocity of the shear wave.

13. Method according to any of claims 6 to 12, wherein the determining step d) further comprises a determination of a transdiaphragmatic pressure based on the variation of the propagation velocity of the shear wave.

14. Method according to any of claims 6 to 13, wherein the stimulation step a) further comprises successive emissions of focused ultrasound waves towards the region of interest, each of said successive emissions being performed:

• according to a different axis, and/or
• according to a different focal length,

the method further comprises a determination of a spatial organization of muscles fascicles based on three-dimensional velocity fields reconstruction.

15. Method according to any of claims 1 to 14, further comprising the step of determining a nerve conduction velocity from a time separating the stimulation of the diaphragm from the occurrence of a movement of the diaphragm associated to said stimulation.

16. Device for measuring one or more diaphragmatic functional parameters of a diaphragm (7) of a human or an animal among a contractility and/or a diaphragmatic pressure and/or a diaphragm function and/or diaphragm effort and/or a diaphragmatic work and/or an identification of the spatial organization of muscle fascicule(s), said device comprising means for processing ultrasound images of one or more moving parts of the diaphragm, said ultrasound images comprising at least 100 images per second of said one or more moving parts of the diaphragm, said means for processing being arranged and/or configured and/or programmed to measure:

• one or more movements of a given part of the diaphragm over time, and/or
• a propagation of a movement from said one or more parts of the diaphragm to one or more adjacent parts over time, and/or
• a propagation speed of a movement from said one or more parts of the diaphragm to one or more adjacent parts over time, and/or
• one or more movements of different parts of the diaphragm over time, and/or
• an amplitude of a movement of one or different parts of the diaphragm over time;

said means for processing being arranged and/or configured and/or programmed to determine one or more functional parameters of the diaphragm from a linear relation between one of the one or more previous measurements being indicative of movement of one or more parts of the diaphragm and the considered functional parameter.

17. Apparatus for measuring one or more diaphragmatic functional parameters of a diaphragm (7) of a human or an animal among a contractility and/or a diaphragmatic pressure and/or a diaphragm function and/or diaphragm effort and/or a diaphragmatic work and/or an identification of the spatial organization of muscle fascicule(s), said apparatus comprising:

- means for stimulating the diaphragm to generate a movement of one or more parts of the diaphragm,
- means for imaging (9) one or more parts of the diaphragm over time, said means for imaging being arranged to:

• emit at least 100 unfocused ultrasound waves per second towards a region of the human or the animal comprising the one or more parts of the diaphragm to be imaged by the imaging means,
• detect ultrasound waves reflected and/or scattered by organic tissues of the human or the animal located in the region,
• process the reflected and/or scattered ultrasound waves over time to generate images

- means for processing images previously acquired, said means for imaging being arranged and/or configured and/or programmed to measure:

• one or more movements of a given part of the diaphragm over time, and/or
• a propagation of a movement from said one or more parts of the diaphragm to one or more adjacent parts over time, and/or
• a propagation speed of a movement from said one or more parts of the diaphragm to one or more adjacent parts over time, and/or
• one or more movements of different parts of the diaphragm over time, and/or
• an amplitude of a movement of one or different parts of the diaphragm over time,
• a time separating the stimulation of the diaphragm from the occurrence of a movement of the diaphragm associated to said stimulation;

said means for processing being arranged and/or configured and/or programmed, based on one or more of those previous measurements, to determine one or more functional parameters of the diaphragm from a linear relation between one of the one or more previous measurements being indicative of movement of one or more parts of the diaphragm and the considered functional parameter.

**Patentansprüche**

1. Verfahren zum Messen eines oder mehrerer Diaphragma-Funktionsparameter eines Diaphragmas (7) eines Menschen oder eines Tieres, darunter einer Kontraktilität und/oder eines Diaphragma-Drucks und/oder einer Diaphragma-Funktion und/oder einer Diaphragma-Anstrengung und/oder einer Diaphragma-Tätigkeit und/oder einer Identifikation der räumlichen Organisation eines Muskelfaszikels/von Muskelfaszikeln, wobei das Verfahren die Schritte umfasst, die bestehen aus:

- a) einem Stimulationsschritt, durch den eine Stimulation des Diaphragmas bereitgestellt wird, um eine Bewegung eines oder mehrerer Teile des Diaphragmas zu erzeugen, wobei der Stimulationsschritt keine assistierte Beatmung oder lebenserhaltenden Maßnahmen umfasst,
- b) während der Bewegung des einen oder der mehreren Teile des Diaphragmas, Abbilden eines oder mehrerer Teile des Diaphragmas im Laufe der Zeit durch einen Bildgebungsschritt, der die Schritte umfasst:

 • Emittieren von mindestens 100 unfokussierten Ultraschallwellen pro Sekunde in Richtung eines Bereichs des Menschen oder des Tieres, der den einen oder die mehreren Teile des Diaphragmas umfasst, die während des Bildgebungsschritts abgebildet werden sollen,
 • Erfassen von Ultraschallwellen, die durch organisches Gewebe des Menschen oder des Tieres, das sich im Bereich befindet, reflektiert und/oder gestreut werden,
 • Verarbeiten der reflektierten und/oder gestreuten Ultraschallwellen im Laufe der Zeit, um durch technische Mittel Bilder zu erzeugen,

- c) Verarbeiten, durch technische Mittel, von Bildern, die zuvor während des Bildgebungsschritts aufgenommen wurden, um zu messen:

 • eine oder mehrere Bewegungen eines bestimmten Teils des Diaphragmas im Laufe der Zeit und/oder
 • eine Ausbreitung einer Bewegung von dem einen oder den mehreren Teilen des Diaphragmas zu einem oder mehreren benachbarten Teilen im Laufe der Zeit und/oder
 • eine Ausbreitungsgeschwindigkeit einer Bewegung von dem einen oder den mehreren Teilen des Diaphragmas zu einem oder mehreren benachbarten Teilen im Laufe der Zeit und/oder
 • eine oder mehrere Bewegungen verschiedener Teile des Diaphragmas im Laufe der Zeit und/oder
 • eine Amplitude einer Bewegung eines oder verschiedener Teile des Diaphragmas im Laufe der Zeit,

- d) basierend auf einer oder mehreren Messungen des Verarbeitungsschritts, Bestimmen, durch technische Mittel, eines oder mehrerer Funktionsparameter des Diaphragmas aus einer linearen Beziehung zwischen einem der in Schritt c) gemessenen Parameter, die eine Bewegung eines oder mehrerer Teile des Diaphragmas angeben, und dem berücksichtigten Funktionsparameter.

2. Verfahren nach Anspruch 1, wobei die Stimulation des Diaphragmas eine elektrische und/oder magnetische Stimulation ist.

3. Verfahren nach Anspruch 2, wobei der Verarbeitungsschritt c) ferner eine Messung der Amplitude der Bewegung des Diaphragmas basierend auf einer Intensität der Stimulation umfasst.

4. Verfahren nach Anspruch 2 oder 3, wobei der Bestimmungsschritt d) ferner eine Bestimmung einer Nervenleitgeschwindigkeit umfasst.

5. Verfahren nach einem der Ansprüche 2 bis 4, wobei der Bestimmungsschritt d) basierend auf einer Ausbreitungsgeschwindigkeit einer Bewegung durch das Diaphragma eine Bestimmung umfasst von:

 - einer Kontraktilität des Diaphragmas und/oder
 - einer lokalen Lähmung des Diaphragmas.

6. Verfahren nach Anspruch 1, wobei:

 - der Stimulationsschritt a) ferner eine mechanische Stimulation und/oder eine akustische Stimulation eines oder mehrerer Teile des Diaphragmas umfasst, wodurch an dem bestimmten Teil eine mechanische Welle und/oder eine akustische Welle erzeugt wird, wobei sich die mechanische Welle und/oder die akustische Welle in Richtung benachbarter Teile des bestimmten Teils ausbreiten,
 - der Bildgebungsschritt b) ferner ein Abbilden des bestimmten Teils und der benachbarten Teile, durch die sich die mechanische Welle und/oder die akustische Welle ausbreitet/ausbreiten, umfasst.

7. Verfahren nach Anspruch 6, wobei die mechanische Welle und/oder die akustische Stimulation eine Ultraschallstimulation sind, wobei die Ultraschallstimulation eine Emission einer oder mehrerer fokussierter Ultraschallwellen pro Sekunde in Richtung eines bestimmten Teils des Diaphragmas umfasst, wobei die eine oder die mehreren fokussierten Ultraschallwellen an dem bestimmten Teil eine elastische Scherwelle erzeugen, wobei sich die elastische Scherwelle in Richtung benachbarter Teile des bestimmten Teils ausbreitet.

8. Verfahren nach Anspruch 6 oder 7, wobei der Verarbeitungsschritt c) ferner eine Messung einer Ausbreitungsgeschwindigkeit der Scherwelle umfasst.

9. Verfahren nach einem der Ansprüche 6 bis 8, wobei der Bestimmungsschritt d) ferner eine Bestimmung einer Kontraktilität des Diaphragmas basierend auf der Ausbreitungsgeschwindigkeit der Scherwelle umfasst.

10. Verfahren nach einem der Ansprüche 6 bis 9, wobei der Stimulationsschritt a) während der Beatmung des Menschen oder des Tieres durchgeführt wird.

11. Verfahren nach einem der Ansprüche 6 bis 10, wobei der Bestimmungsschritt d) ferner eine Bestimmung einer Diaphragma-Tätigkeit umfasst.

12. Verfahren nach einem der Ansprüche 6 bis 11, wobei der Bestimmungsschritt d) ferner eine Bestimmung einer Diaphragma-Aktivität basierend auf der Ausbreitungsgeschwindigkeit der Scherwelle umfasst.

13. Verfahren nach einem der Ansprüche 6 bis 12, wobei der Bestimmungsschritt d) ferner eine Bestimmung eines transdiaphragmatischen Drucks basierend auf der Variation der Ausbreitungsgeschwindigkeit der Scherwelle umfasst.

14. Verfahren nach einem der Ansprüche 6 bis 13, wobei der Stimulationsschritt a) ferner aufeinanderfolgende Emissionen fokussierter Ultraschallwellen in Richtung des interessierenden Bereichs umfasst, wobei jede dieser aufeinanderfolgenden Emissionen durchgeführt wird:

• gemäß einer anderen Achse und/oder
• gemäß einer anderen Brennweite,

wobei das Verfahren ferner eine Bestimmung einer räumlichen Organisation von Muskelfaszikeln basierend auf einer dreidimensionalen Rekonstruktion von Geschwindigkeitsfeldern umfasst.

15. Verfahren nach einem der Ansprüche 1 bis 14, ferner umfassend den Schritt des Bestimmens einer Nervenleitgeschwindigkeit aus einer Zeit, die die Stimulation des Diaphragmas vom Auftreten einer Bewegung des Diaphragmas, die der Stimulation zugeordnet ist, trennt.

16. Vorrichtung zum Messen eines oder mehrerer Diaphragma-Funktionsparameter eines Diaphragmas (7) eines Menschen oder eines Tieres, darunter einer Kontraktilität und/oder eines Diaphragma-Drucks und/oder einer Diaphragma-Funktion und/oder einer Diaphragma-Anstrengung und/oder einer Diaphragma-Tätigkeit und/oder einer Identifikation der räumlichen Organisation eines Muskelfaszikels/von Muskelfaszikeln, wobei die Vorrichtung Mittel zum Verarbeiten von Ultraschallbildern eines oder mehrerer beweglicher Teile des Diaphragmas umfasst, wobei die Ultraschallbilder mindestens 100 Bilder pro Sekunde des einen oder der mehreren beweglichen Teile des Diaphragmas umfassen,

wobei die Mittel zum Verarbeiten angeordnet und/oder konfiguriert und/oder programmiert sind, um zu messen:

• eine oder mehrere Bewegungen eines bestimmten Teils des Diaphragmas im Laufe der Zeit und/oder
• eine Ausbreitung einer Bewegung von dem einen oder den mehreren Teilen des Diaphragmas zu einem oder mehreren benachbarten Teilen im Laufe der Zeit und/oder
• eine Ausbreitungsgeschwindigkeit einer Bewegung von dem einen oder den mehreren Teilen des Diaphragmas zu einem oder mehreren benachbarten Teilen im Laufe der Zeit und/oder
• eine oder mehrere Bewegungen verschiedener Teile des Diaphragmas im Laufe der Zeit und/oder
• eine Amplitude einer Bewegung eines oder verschiedener Teile des Diaphragmas im Laufe der Zeit,

wobei die Mittel zum Verarbeiten angeordnet und/oder konfiguriert und/oder programmiert sind, um einen oder mehrere Funktionsparameter des Diaphragmas aus einer linearen Beziehung zwischen einer der einen oder der mehreren vorherigen Messungen, die eine Bewegung eines oder mehrerer Teile des Diaphragmas angeben, und dem berücksichtigten Funktionsparameter zu bestimmen.

17. Einrichtung zum Messen eines oder mehrerer Diaphragma-Funktionsparameter eines Diaphragmas (7) eines Menschen oder eines Tieres, darunter einer Kontraktilität und/oder eines Diaphragma-Drucks und/oder einer Diaphragma-Funktion und/oder einer Diaphragma-Anstrengung und/oder einer Diaphragma-Tätigkeit und/oder einer Identifikation der räumlichen Organisation eines Muskelfaszikels/von Muskelfaszikeln, wobei die Einrichtung umfasst:

- Mittel zum Stimulieren des Diaphragmas, um eine Bewegung eines oder mehrerer Teile des Diaphragmas zu erzeugen,
- Mittel zum Abbilden (9) eines oder mehrerer Teile des Diaphragmas im Laufe der Zeit, wobei die Mittel zum Abbilden angeordnet sind zum:

• Emittieren von mindestens 100 unfokussierten Ultraschallwellen pro Sekunde in Richtung eines Bereichs des Menschen oder des Tieres, der den einen oder die mehreren Teile des Diaphragmas, die durch die Bildgebungsmittel abgebildet werden sollen, umfasst,

• Erfassen von Ultraschallwellen, die durch organisches Gewebe des Menschen oder des Tieres, das sich im Bereich befindet, reflektiert und/oder gestreut werden,

• Verarbeiten der reflektierten und/oder gestreuten Ultraschallwellen im Laufe der Zeit, um Bilder zu erzeugen,

- Mittel zum Verarbeiten zuvor aufgenommener Bilder, wobei die Mittel zum Abbilden angeordnet und/oder konfiguriert und/oder programmiert sind, um zu messen:

• eine oder mehrere Bewegungen eines bestimmten Teils des Diaphragmas im Laufe der Zeit und/oder

• eine Ausbreitung einer Bewegung von dem einen oder den mehreren Teilen des Diaphragmas zu einem oder mehreren benachbarten Teilen im Laufe der Zeit und/oder

• eine Ausbreitungsgeschwindigkeit einer Bewegung von dem einen oder den mehreren Teilen des Diaphragmas zu einem oder mehreren benachbarten Teilen im Laufe der Zeit und/oder

• eine oder mehrere Bewegungen verschiedener Teile des Diaphragmas im Laufe der Zeit und/oder

• eine Amplitude einer Bewegung eines oder verschiedener Teile des Diaphragmas im Laufe der Zeit,

• eine Zeit, die die Stimulation des Diaphragmas vom Auftreten einer Bewegung des Diaphragmas, die der Stimulation zugeordnet ist, trennt;

wobei die Mittel zum Verarbeiten angeordnet und/oder konfiguriert und/oder programmiert sind, um basierend auf einer oder mehreren dieser vorherigen Messungen einen oder mehrere Funktionsparameter des Diaphragmas aus einer linearen Beziehung zwischen einer der einen oder der mehreren vorherigen Messungen, die eine Bewegung eines oder mehrerer Teile des Diaphragmas angeben, und dem berücksichtigten Funktionsparameter zu bestimmen.

**Revendications**

1. Procédé de mesure d'un ou plusieurs paramètres fonctionnels diaphragmatiques d'un diaphragme (7) d'un homme ou d'un animal parmi une contractilité et/ou une pression diaphragmatique et/ou une fonction diaphragmatique et/ou un effort diaphragmatique et/ou un travail diaphragmatique et/ou une identification de l'organisation spatiale de fascicule(s) musculaire(s), ledit procédé comprenant les étapes consistant à :

- a) une étape de stimulation par laquelle une stimulation du diaphragme est fournie pour générer un mouvement d'une ou plusieurs parties du diaphragme, ladite étape de stimulation ne comprend pas de ventilation assistée ou de ventilation artificielle,

- b) pendant le mouvement de ladite ou desdites parties du diaphragme, l'imagerie d'une ou de plusieurs parties du diaphragme au fil du temps à travers une étape d'imagerie comprenant les étapes consistant à :

• l'émission d'au moins 100 ondes ultrasonores non focalisées par seconde vers une région de l'homme ou de l'animal comprenant la ou les parties du diaphragme à imager au cours de l'étape d'imagerie,

• la détection des ondes ultrasonores réfléchies et/ou diffusées par les tissus organiques de l'homme ou de l'animal situés dans la région,

• le traitement des ondes ultrasonores réfléchies et/ou diffusées au fil du temps pour générer des images par des moyens techniques,

- c) le traitement, par des moyens techniques, des images acquises précédemment au cours de l'étape d'imagerie pour mesurer :

• un ou plusieurs mouvements d'une partie donnée du diaphragme au fil du temps, et/ou

• la propagation d'un mouvement de ladite ou desdites parties du diaphragme à une ou plusieurs parties adjacentes au fil du temps, et/ou

• une vitesse de propagation d'un mouvement de ladite ou desdites parties du diaphragme à une ou plusieurs parties adjacentes au fil du temps, et/ou

• un ou plusieurs mouvements de différentes parties du diaphragme au fil du temps, et/ou

• une amplitude d'un mouvement d'une ou de plusieurs parties du diaphragme au fil du temps

- d) sur la base d'une ou plusieurs mesures de l'étape de traitement, la détermination, par des moyens techniques, d'un ou plusieurs paramètres fonctionnels du diaphragme à partir d'une relation linéaire entre l'un des paramètres mesurés à l'étape c) indiquant un mouvement d'une ou plusieurs parties du diaphragme et du paramètre fonctionnel considéré.

2. Procédé selon la revendication 1, dans lequel la stimulation du diaphragme est une stimulation électrique et/ou magnétique.

3. Procédé selon la revendication 2, dans lequel l'étape de traitement c) comprend en outre une mesure de l'amplitude du mouvement du diaphragme sur la base d'une intensité de la stimulation.

4. Procédé selon la revendication 2 ou 3, dans lequel l'étape de détermination d) comprend en outre une détermination d'une vitesse de conduction nerveuse.

5. Procédé selon l'une quelconque des revendications 2 à 4, dans lequel, sur la base d'une vitesse de propagation d'un mouvement à travers le diaphragme, l'étape de détermination d) comprend une détermination de :

    - une contractilité du diaphragme, et/ou
    - une paralysie localisée du diaphragme.

6. Procédé selon la revendication 1, dans lequel :

    - l'étape de stimulation a) comprend en outre une stimulation mécanique et/ou une stimulation acoustique d'une ou plusieurs parties du diaphragme générant une onde mécanique et/ou une onde acoustique au niveau de ladite partie donnée, l'onde mécanique et/ou l'onde acoustique se propageant vers les parties adjacentes de ladite partie donnée,
    - l'étape d'imagerie b) comprend en outre l'imagerie de ladite partie donnée et desdites parties adjacentes à travers lesquelles l'onde mécanique et/ou l'onde acoustique se propage(nt).

7. Procédé selon la revendication 6, dans lequel l'onde mécanique et/ou la stimulation acoustique est une stimulation ultrasonique, ladite stimulation ultrasonique comprenant une émission d'une ou plusieurs ondes ultrasoniques focalisées par seconde vers une partie donnée du diaphragme, ladite ou lesdites ondes ultrasoniques focalisées générant une onde de cisaillement élastique au niveau de ladite partie donnée, l'onde de cisaillement élastique se propageant vers les parties adjacentes de ladite partie donnée.

8. Procédé selon la revendication 6 ou 7, dans lequel l'étape de traitement c) comprend en outre une mesure d'une vitesse de propagation de l'onde de cisaillement.

9. Procédé selon l'une quelconque des revendications 6 à 8, dans lequel l'étape de détermination d) comprend en outre une détermination d'une contractilité du diaphragme sur la base de la vitesse de propagation de l'onde de cisaillement.

10. Procédé selon l'une quelconque des revendications 6 à 9, dans lequel l'étape de stimulation a) est réalisée pendant la ventilation de l'homme ou de l'animal.

11. Procédé selon l'une quelconque des revendications 6 à 10, dans lequel l'étape de détermination d) comprend en outre une détermination d'un travail du diaphragme.

12. Procédé selon l'une quelconque des revendications 6 à 11, dans lequel l'étape de détermination d) comprend en outre une détermination d'une activité du diaphragme sur la base de la vitesse de propagation de l'onde de cisaillement.

13. Procédé selon l'une quelconque des revendications 6 à 12, dans lequel l'étape de détermination d) comprend en outre une détermination d'une pression transdiaphragmatique sur la base de la variation de la vitesse de propagation de l'onde de cisaillement.

14. Procédé selon l'une quelconque des revendications 6 à 13, dans lequel l'étape de stimulation a) comprend en outre des émissions successives d'ondes ultrasonores focalisées vers la région d'intérêt, chacune desdites émissions successives étant réalisée :

    • selon un axe différent, et/ou
    • selon une longueur focale différente,

le procédé comprend en outre une détermination d'une organisation spatiale des fascicules musculaires sur la base d'une reconstruction tridimensionnelle des champs de vitesse.

15. Procédé selon l'une quelconque des revendications 1 à 14, comprenant en outre l'étape consistant à la détermination d'une vitesse de conduction nerveuse à partir d'un temps séparant la stimulation du diaphragme de l'apparition d'un mouvement du diaphragme associé à ladite stimulation.

**16.** Procédé de mesure d'un ou plusieurs paramètres fonctionnels diaphragmatiques d'un diaphragme (7) d'un homme ou d'un animal parmi une contractilité et/ou une pression diaphragmatique et/ou une fonction diaphragmatique et/ou un effort diaphragmatique et/ou un travail diaphragmatique et/ou une identification de l'organisation spatiale de fascicule(s) musculaire(s), ledit dispositif comprenant des moyens de traitement d'images ultrasonores d'une ou plusieurs parties mobiles du diaphragme, lesdites images ultrasonores comprenant au moins 100 images par seconde de ladite ou desdites parties mobiles du diaphragme,

lesdits moyens de traitement étant agencés et/ou configurés et/ou programmés pour mesurer :

• un ou plusieurs mouvements d'une partie donnée du diaphragme au fil du temps, et/ou
• la propagation d'un mouvement de ladite ou desdites parties du diaphragme à une ou plusieurs parties adjacentes au fil du temps, et/ou
• une vitesse de propagation d'un mouvement de ladite ou desdites parties du diaphragme à une ou plusieurs parties adjacentes au fil du temps, et/ou
• un ou plusieurs mouvements de différentes parties du diaphragme au fil du temps, et/ou
• une amplitude d'un mouvement d'une ou de plusieurs parties du diaphragme au fil du temps ;

lesdits moyens de traitement étant agencés et/ou configurés et/ou programmés pour déterminer un ou plusieurs paramètres fonctionnels du diaphragme à partir d'une relation linéaire entre l'une parmi la ou les mesures précédentes indiquant un mouvement d'une ou plusieurs parties du diaphragme et le paramètre fonctionnel considéré.

**17.** Appareil de mesure d'un ou plusieurs paramètres fonctionnels diaphragmatiques d'un diaphragme (7) d'un homme ou d'un animal parmi une contractilité et/ou une pression diaphragmatique et/ou une fonction diaphragmatique et/ou un effort diaphragmatique et/ou un travail diaphragmatique et/ou une identification de l'organisation spatiale de fascicule(s) musculaire(s), ledit appareil comprenant :

- des moyens de stimulation du diaphragme pour générer un mouvement d'une ou plusieurs parties du diaphragme,
- des moyens d'imagerie (9) d'une ou plusieurs parties du diaphragme au fil du temps, lesdits moyens d'imagerie étant agencés pour :

• émettre au moins 100 ondes ultrasonores non focalisées par seconde vers une région de l'homme ou de l'animal comprenant la ou les parties du diaphragme à imager par les moyens d'imagerie,
• détecter des ondes ultrasonores réfléchies et/ou diffusées par les tissus organiques de l'homme ou de l'animal situés dans la région,
• traiter des ondes ultrasonores réfléchies et/ou diffusées au fil du temps pour générer des images

- des moyens de traitement des images précédemment acquises, lesdits moyens d'imagerie étant agencés et/ou configurés et/ou programmés pour mesurer :

• un ou plusieurs mouvements d'une partie donnée du diaphragme au fil du temps, et/ou
• la propagation d'un mouvement de ladite ou desdites parties du diaphragme à une ou plusieurs parties adjacentes au fil du temps, et/ou
• une vitesse de propagation d'un mouvement de ladite ou desdites parties du diaphragme à une ou plusieurs parties adjacentes au fil du temps, et/ou
• un ou plusieurs mouvements de différentes parties du diaphragme au fil du temps, et/ou
• une amplitude d'un mouvement d'une ou de plusieurs parties du diaphragme au fil du temps,
• un temps séparant la stimulation du diaphragme de l'apparition d'un mouvement du diaphragme associé à ladite stimulation ;

lesdits moyens de traitement étant agencés et/ou configurés et/ou programmés, sur la base d'une ou plusieurs de ces mesures précédentes, pour déterminer un ou plusieurs paramètres fonctionnels du diaphragme à partir d'une relation linéaire entre l'une parmi la ou les mesures précédentes indiquant un mouvement d'une ou plusieurs parties du diaphragme et le paramètre fonctionnel considéré.

1
3
4
2

5
7
8
6

9

Fig. 1

Fig. 3

Electrical or magnetic phrenic stimulation

Pes

0.1 s

Pga

Pdi (if bilateral magnetic or electrical stimulation)

EMG (if unilateral electrical stimulation)

Diaphragm displacement

Fig. 2

Fig. 4

Fig. 5

Fig.6

time (s)

Fig.7

Fig. 9

Fig. 10

Fig. 8

Fig. 11

**EP 3 849 413 B1**

**REFERENCES CITED IN THE DESCRIPTION**

**Non-patent literature cited in the description**

- **G H MILLS et al.** Unilateral magnetic stimulation of the phrenic nerve. *Thorax,* 01 November 1995, vol. 50, 1162-1172